(19) Europäisches Patentamt
European Patent Office
Office européen des brevets

(11) **EP 2 610 219 B1**

(12) **EUROPEAN PATENT SPECIFICATION**

(45) Date of publication and mention
of the grant of the patent:
**11.10.2017  Bulletin 2017/41**

(51) Int Cl.:
*C01B 33/18* *(2006.01)*    *C01B 37/00* *(2006.01)*
*C01B 39/08* *(2006.01)*

(21) Application number: **13155806.6**

(22) Date of filing: **12.03.2007**

(54) **Process for the preparation of a pyrogenic silicon-titanium mixed oxide powder**

Verfahren zur Herstellung eines pyrogenen Silicium-Titan gemischten Oxidpulvers

Procédé pour la préparation d'une poudre d'oxyde pyrogène mixte de titane et silicium

(84) Designated Contracting States:
**AT BE BG CH CY CZ DE DK EE ES FI FR GB GR HU IE IS IT LI LT LU LV MC MT NL PL PT RO SE SI SK TR**

(30) Priority:  **15.04.2006  DE 102006017701**

(43) Date of publication of application:
**03.07.2013  Bulletin 2013/27**

(62) Document number(s) of the earlier application(s) in accordance with Art. 76 EPC:
**07712508.6 / 2 007 678**

(73) Proprietor: **Evonik Degussa GmbH**
**45128 Essen (DE)**

(72) Inventors:
• **Schumacher, Kai**
  **65719 Hofheim (DE)**
• **Mörters, Martin**
  **Mobile, AL 36695 (US)**
• **Mangold, Helmut**
  **63517 Rodenbach (DE)**
• **Hasenzahl, Steffen**
  **63452 Hanau (DE)**

(56) References cited:
**EP-A1- 0 814 058    US-A1- 2003 129 153**

## Description

The invention relates to a process for the preparation of pyrogenic silicon-titanium mixed oxide powder.

The use of silicon-titanium mixed oxide powders for the preparation of titanium-containing zeolites is known from EP-A-814058. Titanium-containing zeolites are efficient catalysts for the oxidation of olefins using hydrogen peroxide. They are obtained by hydrothermal synthesis starting from silicon-titanium mixed oxide powders in the presence of a template. In EP-A-814058, it is disclosed that pyrogenic silicon-titanium mixed oxides having a silicon dioxide content of 75 to 99.9% by weight and a titanium dioxide content of 0.1 to 25% by weight can be employed for this. A composition which contains from 90 to 99.5% by weight of silicon dioxide and 0.5 to 5% by weight of titanium dioxide is particularly advantageous. As templates, amines, ammonium compounds or alkali/alkaline earth metal hydroxides can be employed.

A disadvantage of the process disclosed in EP-A-814058 is the long reaction time which is necessary for the reaction of the silicon-titanium mixed oxide in the presence of the template. Furthermore, not all titanium-containing zeolites obtained according to EP-A-814058 show adequate catalytic activity.

An object of the invention was therefore to make available a silicon-titanium mixed oxide, with which the reaction times in the preparation of the titanium-containing zeolite can be reduced.

The invention relates to a process for the preparation of a pyrogenic silicon-titanium mixed oxide powder having

- a BET surface area is 200 to 400 $m^2$/g,
- a silicon dioxide content of 97.0 $\pm$ 1.5% by weight,
- a titanium dioxide content of 3.5 $\pm$ 1.0% by weight and
- the sum of silicon dioxide content and titanium dioxide content is greater than 99.7% by weight,

all percentages by weight relating to the total amount of the powder,
in which

- 97.0 $\pm$ 1.5 parts by weight calculated as $SiO_2$ of silicon halide and 3.5 $\pm$ 1.5 parts by weight calculated as $TiO_2$ of titanium halide are evaporated, the vapours are taken to a mixing chamber,

- hydrogen and primary air are taken to the mixing chamber separately therefrom,

- the mixture of the vapours of silicon halide and titanium halide, hydrogen-containing combustible gas and primary air is subsequently ignited in a burner and the flame is burned into a reaction chamber,

- secondary air is additionally introduced into the reaction chamber, the solid is subsequently separated from gaseous substances, and

- the solid is subsequently freed as far as possible from halide-containing substances by treatment with steam at temperatures of 250 to 700°C

<u>wherein</u>

- the amount of the required substances consisting of silicon chloride, titanium chloride, combustible gas, primary air and secondary air being chosen such that an adiabatic flame temperature $T_{ad}$ results, for which the following is true:

$$900°C < T_{ad} < 1200°C,$$

with $T_{ad}$ = temperature of required substances + sum of the reaction enthalpies of the partial reactions/heat capacity of the substances which leave the reaction chamber, comprising silicon dioxide, water, hydrogen chloride, if appropriate carbon dioxide, oxygen, nitrogen, and if appropriate of the carrier gas if this is not air or nitrogen, the specific heat capacity of these substances at 1000°C being used as a basis.

The specific heat capacities can be determined, for example, with the aid of the VDI Wärmeatlas [VDI heat atlas] (Chapter 7.1 to 7.3 and 3.7, 8th Edition).

The reaction of the silicon chlorides and titanium chlorides in the presence of oxygen and of a combustible gas yields silicon-titanium mixed oxide, water, hydrochloric acid and, in the case of carbon-containing silicon and/or titanium compounds and/or carbon-containing combustible gases, carbon dioxide. The reaction enthalpies of these reactions can be calculated by means of standard works known to the person skilled in the art.

In Table 1, some selected values of reaction enthalpies of the reaction of silicon halides and titanium tetrachloride in the presence of hydrogen and oxygen are given.

Methyltrichlorosilane (MTCS, $CH_3SiCl_3$), trichlorosilane (TCS, $SiHCl_3$) and/or dichlorosilane (DCS, $SiH_2Cl_2$) and titanium tetrachloride can particularly preferably be employed.

**Table 1: Reaction enthalpies**

|  | KJ/mol |
|---|---|
| $H_2$ | -241.8 |
| $SiCl_4$ | -620.1 |
| $SiHCl_3$ | -659.4 |
| $SiH_2Cl_2$ | -712.3 |
| $C_3H_7SiCl_3$ | -2700.2 |
| $CH_3SiCl_3$ | -928.3 |
| $(CH_3)_3SiCl$ | -2733.8 |
| $TiCl_4$ | -553.4 |

Suitable combustible gases are hydrogen, methane, ethane, propane and/or natural gas, hydrogen being preferred.

It can further be advantageous if the exit velocity of the reaction mixture from the mixing chamber to the reaction space is 10 to 80 m/s.

The vapours of the silicon chloride and of the titanium chloride can also be taken to the mixing chamber, in mixed or separate form, by means of a carrier gas.

The required substances combustible gas, primary air and/or secondary air can be introduced in preheated form. A suitable temperature range is 50 to 400°C.

Furthermore, primary and/or secondary air can be enriched with oxygen.

Preferably, the process according to the invention can be carried out such that $SiCl_4$ is employed as silicon halide, $TiCl_4$ is employed as titanium halide and the adiabatic flame temperature $T_{ad}$ = 1050 $\pm$ 50°C.

Pyrogenic is to be understood as meaning metal mixed oxide particles obtained by flame oxidation and/or flame hydrolysis. In this process, oxidizable and/or hydrolysable starting substances are as a rule oxidized or hydrolysed in a hydrogen-oxygen flame. The metal mixed oxide particles according to the invention are as far as possible pore-free and have free hydroxyl groups on the surface. They are present in the form of aggregated primary particles.

It has been shown that a high BET surface area markedly reduces the period of time for the preparation of a titanium-containing zeolite from the silicon-titanium mixed oxide powder obtained by the process of the invention.

A silicon-titanium mixed oxide powder having a BET surface area of 250 to 350 $m^2$/g is preferred and particularly preferably one of 300 $\pm$ 30 $m^2$/g.

Furthermore, a silicon-titanium mixed oxide powder having a silicon dioxide content of 97.0 $\pm$ 1.0% by weight and a titanium dioxide content of 3.5 $\pm$ 0.75% by weight is preferred where the sum of silicon dioxide content and titanium dioxide content is greater than 99.9% by weight. A silicon-titanium mixed oxide powder having a silicon dioxide content of 97.0 $\pm$ 0.5% by weight and a titanium dioxide content of 3.5 $\pm$ 0.5% by weight is particularly preferred where the sum of silicon dioxide content and titanium dioxide content is greater than 99.9% by weight.

The sum of silicon dioxide content and titanium dioxide content in the powder obtained by the process according to the invention is greater than 99.7% by weight and preferably greater than 99.9% by weight. The content of the metals Al, Ca, Co, Fe, K, Na, Ni and Zn is preferably less than 50 ppm each and particularly preferably less than 25 ppm each. The content of chloride is preferably less than 700 ppm. It has proved advantageous for the preparation of titanium-containing zeolites if the contents of these metals and chloride do not exceed these values. These impurities can originate from the required substances and/or can be caused due to the process.

A further subject is a dispersion which comprises the silicon-titanium mixed oxide powder obtained by the process according to the invention and water.

The average aggregate diameter of the silicon-titanium mixed oxide particles in the dispersion is preferably less than 200 nm and particularly preferably less than 100 nm.

Preferably, the following is true for the dispersion according to the invention: 10 $\leq$ mol of water/mol of silicon-titanium mixed oxide $\leq$ 20. Particularly preferably, the range is 12 $\leq$ mol of water/mol of silicon-titanium mixed oxide $\leq$ 17.

Furthermore, a dispersion can be preferred which additionally contains a basic, quaternary ammonium compound. Dispersions are particularly preferred which contain tetraalkylammonium hydroxides such as, for example, tetraethylammonium hydroxide, tetra-n-propylammonium hydroxide and/or tetra-n-butylammonium hydroxide.

The content of quaternary, basic ammonium compound in the dispersion according to the invention is not limited. If the dispersion is to be stored for a relatively long time, it can be advantageous to add to it only a part of the amount of the dispersion necessary for the preparation of a titanium-containing zeolite. Preferably, the quaternary, basic ammonium compound can be added in such an amount that a pH of 9 to 11, in particular 9.5 to 10.5, results. The dispersion shows good stability in this pH range.

If the dispersion is to be employed, for example, immediately after its preparation for the preparation of a titanium-containing zeolite, the dispersion can already also contain the total amount of quaternary, basic ammonium compound. Preferably, the following is then true: $0.12 \leq$ mol of ammonium compound/mol of silicon-titanium mixed oxide $< 0.20$, $0.13 \leq$ mol of ammonium compound/mol of silicon-titanium mixed oxide $\leq 0.17$ being particularly preferred.

The process for the preparation of the dispersion comprises the steps:

- water, which, if the silicon-titanium mixed oxide powder introduced later leads to a pH of the aqueous phase of <2 or >4, is adjusted by addition of acids or bases to pHs of 2 to 4, is recycled from a receiver by means of a rotor/stator machine, and

- an amount of the silicon-titanium mixed oxide powder is introduced continuously or batchwise by means of a filling device and with the rotor/stator machine running into the shear zone between the slots of the rotor teeth and of the stator slots such that a predispersion having a solids content of 20 to 40% by weight results, and

- after all the silicon-titanium mixed oxide powder has been added, the filling device is closed and the predispersion is sheared further such that the shear rate lies in the range between 10 000 and 40 000 s$^{-1}$, and

- if appropriate water and a basic, quaternary ammonium compound are subsequently added with retention of the dispersion conditions.

The silicon-titanium mixed oxide powder prepared by the process according to the invention and a basic, quaternary ammonium compound can be used for the preparation of a titanium-containing zeolite by treatment in an aqueous medium at a temperature of 150 to 220°C for a period of less than 12 hours.

Preferably, the process is carried out such that the following is true: $10 \leq$ mol of water/mol of silicon-titanium mixed oxide $\leq 20$. Particularly preferably, the range is $12 \leq$ mol of water/mol of silicon-titanium mixed oxide $\leq 17$.

It is furthermore advantageous to carry out the process such that the following is true: $0.12 \leq$ mol of ammonium compound/mol of silicon-titanium mixed oxide $< 0.20$. Particularly preferably, the range is $0.13 \leq$ mol of ammonium compound/mol of silicon-titanium mixed oxide $\leq 0.16$.

As basic, quaternary ammonium compounds, tetraalkylammonium hydroxides such as, for example, tetraethylammonium hydroxide, tetra-n-propylammonium hydroxide and/or tetra-n-butylammonium hydroxide are particularly preferred.

Basic, quaternary ammonium compounds are used as templates which determine the crystal structure by incorporation into the crystal lattice. Tetra-n-propylammonium hydroxide is preferably employed for the preparation of titanium silicalite-1 (MFI structure), tetra-n-butylammonium hydroxide for the preparation of titanium silicalite-2 (MEL structure) and tetraethylammonium hydroxide for the preparation of titanium $\beta$-zeolites (BEA crystal structure).

Also the dispersion comprising the silicon-titanium mixed oxide obtained by the process according to the invention, if appropriate with further addition of a basic, quaternary ammonium compound may be used for the for the preparation of a titanium-containing zeolite, by treatment at a temperature of 150 to 220°C for a period of less than 12 hours.

Under the specified conditions of the process, the crystallization time is conventionally less than 12 hours. The crystals are separated by filtering, centrifuging or decanting and washed with a suitable washing liquid, preferably water. The crystals are then dried if needed and calcined at a temperature between 400°C and 1000°C, preferably between 500°C and 750°C in order to remove the template.

The particle fineness of less than 200 nm in the dispersion leads to rapid dissolution of the particles and formation of the titanium-containing zeolite.

**Examples:**

Required materials: The required materials silicon tetrachloride and titanium tetrachloride of Examples 1 to 5 have contents of Na, K, Fe, Co, Ni, Al, Ca and Zn of < 50 ppm.

Examples 1 to 5: Titanium-silicon mixed oxide powder

Example 1: 5.15 kg/h of silicon tetrachloride and 0.15 kg/h of titanium tetrachloride are evaporated. The vapours are taken to a mixing chamber by means of 15 Nm$^3$/h of nitrogen as a carrier gas. Separately therefrom, 2 Nm$^3$/h of hydrogen and 8 Nm$^3$/h of primary air are introduced into the mixing chamber. The reaction mixture is fed to a burner and ignited in a central tube. The flame burns here in a water-cooled flame tube. 15 Nm$^3$/h of secondary air are additionally introduced into the reaction space. The resulting powder is separated in a filter connected in series and subsequently treated with water vapour at 520°C in countercurrent.

Examples 2-4 are carried out analogously to Example 1 using the amounts listed in Table 2.

Example 5 is a comparative example whose composition lies in the range claimed, but has a markedly lower BET surface area.

The substance parameters of the powders obtained are summarized in Table 2.

In all examples, the content of Na is <10 ppm, K <10 ppm, Fe ≤1 ppm, Co <1 ppm, Ni<1 ppm, Al <10 ppm, Ca <10 ppm, Zn <10 ppm.

### table 2: Required substances and amounts, analytical values of the silicon-titanium mixed oxide powders

| Example | | 1 | 2 | 3 | 4 | 5 |
|---|---|---|---|---|---|---|
| SiCl$_4$ | kg/h | 5.15 | 8.0 | 8.0 | 5.15 | 5.15 |
| TiCl$_4$ | kg/h | 0.15 | 0.21 | 0.21 | 0.15 | 0.15 |
| H$_2$ core | Nm$^3$/h | 2.0 | 3.0 | 3.4 | 2.10 | 3.50 |
| H$_2$ jacket | Nm$^3$/h | 1.0 | 0.5 | 0.5 | 1.0 | 1.0 |
| Primary air | Nm$^3$/h | 8.0 | 10.7 | 10.0 | 12.5 | 10.0 |
| Secondary air | Nm$^3$/h | 15.0 | 15.0 | 15.0 | 15.0 | 15.0 |
| Tad | °C | 1026 | 1059 | 1160 | 930 | 1275 |
| VBr | m/s | 32 | 30 | 21 | 33 | 31 |
| BET | m$^2$/g | 312 | 315 | 203 | 375 | 80 |
| SiO$_2$ | % by wt | 96.4 | 96.8 | 96.4 | 96.6 | 96.6 |
| TiO$_2$ | % by wt | 3.4 | 3.0 | 3.5 | 3.3 | 3.4 |

Example 6: Preparation of a dispersion

32.5 kg of completely demineralized water are initially introduced into a 100 l stainless steel make-up vessel. Subsequently, with the aid of the suction nozzle of the Ystral Conti-TDS 4 (stator slots: 6 mm ring and 1 mm ring, rotor/stator distance about 1 mm), 17.5 kg of the silicon-titanium mixed oxide powder from Example 1 are drawn in under shear conditions. After completion of the drawing-in, the suction nozzle is closed and the 35 per cent by weight pre-dispersion is subsequently additionally sheared at 3000 rpm for 10 min. Undesired warming of the dispersion due to the high energy input is countered by a heat exchanger and the temperature increase is restricted to a maximum of 40°C. Due to the acidic character of the pyrogenically prepared silicon-titanium mixed oxide powder, the pH of the dispersion is about 3.6.

Subsequently, 28.6 kg of completely demineralized water are added and a pH of 10.0 is rapidly adjusted with intensive shearing and thorough mixing using 1.0 kg of tetra-n-propylammonium hydroxide solution (40% by weight in water).

The dispersion has the following values:

water/silicon-titanium mixed oxide 11.7
average aggregate diameter 92 nm (determined with Horiba LA 910)

Example 7: Preparation of a titanium-containing zeolite starting from silicon-titanium mixed oxide powder

137.0 g of a tetra-n-propylammonium hydroxide solution (40% by weight in water) and 434.2 g of deionized water are initially introduced into a polyethylene beaker and 111.1 g of the pyrogenic silicon-titanium mixed oxide powder from Example 1 are incorporated with intensive stirring. The resulting gel is initially aged for 2 hours at 80°C with intensive stirring and subsequently crystallized in an autoclave at 180°C for 10 hours. The solid obtained is separated from the mother liquor by centrifuging, washed three times with 250 ml each of deionized water, dried at 90°C and calcined in an air atmosphere for 4 hours at 550°C.

Water/silicon-titanium mixed oxide 13.1

Tetrapropylammonium hydroxide/silicon-titanium mixed oxide 0.15

Example 8 is carried out analogously to Example 7 but using the silicon-titanium mixed oxide powder from Example 5. The incorporation of the powder manifestly needs more time than in Example 7.

Example 9: Preparation of a titanium-containing zeolite starting from a dispersion comprising silicon-titanium mixed oxide powder

505 g of the dispersion from Example 6, 46.7 g of deionized $H_2O$ and 130.6 g of a tetra-n-propylammonium hydroxide solution (40% by weight in water) are initially introduced into a polyethylene beaker and initially aged for four hours at 80°C with stirring and subsequently crystallized in an autoclave at 180°C for 10 hours. The solid obtained is separated from the mother liquor by centrifuging, washed three times with 250 ml each of deionized water, dried at 90°C and calcined in an air atmosphere for four hours at 550°C.

Water/silicon-titanium mixed oxide 13.2

Tetrapropylammonium hydroxide/silicon-titanium mixed oxide 0.14

The X-ray diffractogram of the crystals obtained from Examples 7 to 9 shows the diffraction pattern typical for the MFI structure; the IR spectrum shows the characteristic band at 960 cm$^{-1}$. The UV-vis spectrum shows that the sample is free of titanium dioxide and titanates.

In the epoxidation of propylene using aqueous hydrogen peroxide solution, the following is true for the catalytic activity of the titanium silicalites obtained from Examples 7, 8 and 9:

$$9 > 7 >> 8.$$

## Claims

1.  Process for the preparation of a pyrogenic silicon-titanium mixed oxide powder having

    - a BET surface area is 200 to 400 m$^2$/g,
    - a silicon dioxide content of $97.0 \pm 1.5$% by weight,
    - a titanium dioxide content of $3.5 \pm 1.0$% by weight and
    - the sum of silicon dioxide content and titanium dioxide content is greater than 99.7% by weight,

    all percentages by weight relating to the total amount of the powder, in which

    - $97.0 \pm 1.5$ parts by weight calculated as $SiO_2$ of silicon halide and $3.5 \pm 1.5$ parts by weight calculated as $TiO_2$ of titanium halide are evaporated, the vapours are taken to a mixing chamber,
    - hydrogen and primary air are taken to the mixing chamber separately therefrom,
    - the mixture of the vapours of silicon halide and titanium halide, hydrogen-containing combustible gas and primary air is subsequently ignited in a burner and the flame is burned into a reaction chamber,
    - secondary air is additionally introduced into the reaction chamber, the solid is subsequently separated from gaseous substances, and
    - the solid is subsequently freed as far as possible from halide-containing substances by treatment with steam at temperatures of 250 to 700°C

    **characterized in that**

    - the amount of the required substances consisting of silicon chloride, titanium chloride, combustible gas, primary air and secondary air being chosen such that an adiabatic flame temperature $T_{ad}$ results, for which the following is true:

$$900°C < T_{ad} < 1200°C,$$

with $T_{ad}$ = temperature of required substances + sum of the reaction enthalpies of the partial reactions/heat capacity of the substances which leave the reaction chamber, comprising silicon dioxide, water, hydrogen chloride, if appropriate carbon dioxide, oxygen, nitrogen, and if appropriate of the carrier gas if this is not air or nitrogen, the specific heat capacity of these substances at 1000°C being used as a basis.

**2.** Process according to Claim 1,
**characterized in that**
$SiCl_4$ is employed as silicon halide, $TiCl_4$ is employed as titanium halide and

$$T_{ad} = 1050 ± 50°C.$$

**3.** Process according to Claim 1 or 2,
**characterized in that**
the exit velocity $v_{Br}$ of the gases employed from the burner into the reaction space is 10 to 80 m/s.

**Patentansprüche**

**1.** Verfahren zur Herstellung eines pyrogenen Silicium-Titan-Mischoxidpulvers, das

- eine BET-Oberfläche von 200 bis 400 $m^2/g$,
- einen Siliciumdioxid-Anteil von 97,0 $\pm$ 1,5 Gew.-%,
- einen Titandioxid-Anteil von 3,5 $\pm$ 1,0 Gew.-% aufweist und
- wobei die Summe aus Siliciumdioxid-Anteil und Titandioxid-Anteil größer als 99,7 Gew.-% ist,

wobei sich alle Gewichtsprozentangaben auf die Gesamtmenge des Pulvers beziehen, in dem

- 97,0 $\pm$ 1,5 als $SiO_2$ berechnete Gewichtsanteile Siliciumhalogenid und 3, 5 $\pm$ 1, 5 als $TiO_2$ berechnete Gewichtsanteile Titanhalogenid verdampft werden, die Dämpfe in eine Mischkammer überführt werden,
- getrennt hiervon Wasserstoff und Primärluft in die Mischkammer überführt werden,
- anschließend das Gemisch aus den Dämpfen von Siliciumhalogenid und Titanhalogenid, wasserstoffhaltigem Brenngas und Primärluft in einem Brenner gezündet wird und die Flamme in eine Reaktionskammer hinein verbrannt wird,
- zusätzlich Sekundärluft in die Reaktionskammer eingebracht wird, anschließend der Feststoff von gasförmigen Stoffen abgetrennt wird, und
- nachfolgend der Feststoff durch eine Behandlung mit Wasserdampf bei Temperaturen von 250 bis 700°C von halogenidhaltigen Substanzen weitestgehend befreit wird,

**dadurch gekennzeichnet, dass**

- die Menge der Einsatzstoffe bestehend aus Siliciumchlorid, Titanchlorid, Brenngas, Primärluft und Sekundärluft so gewählt ist, dass eine adiabate Flammentemperatur $T_{ad}$ resultiert, für die gilt

$$900°C < T_{ad} < 1200°C,$$

mit
$T_{ad}$ = Temperatur Einsatzstoffe + Summe der Reaktionsenthalpien der Teilreaktionen / Wärmekapazität der Stoffe, die die Reaktionskammer verlassen, umfassend Siliciumdioxid, Wasser, Chlorwasserstoff, gegebenenfalls Kohlendioxid, Sauerstoff, Stickstoff, und gegebenenfalls des Traggases, wenn dieses nicht Luft oder Stickstoff ist, wobei die spezifische Wärmekapazität dieser Stoffe bei 1000°C zugrunde gelegt wird.

**2.** Verfahren nach Anspruch 1,

**dadurch gekennzeichnet, dass**

als Siliciumhalogenid $SiCl_4$, als Titanhalogenid $TiCl_4$ eingesetzt wird und

$$T_{ad} = 1050 \pm 50°C.$$

**3.** Verfahren nach den Ansprüchen 1 oder 2,
**dadurch gekennzeichnet, dass**
die Ausströmgeschwindigkeit $v_{Br}$ der eingesetzten Gase aus dem Brenner in den Reaktionsraum 10 bis 80 m/s ist.

**Revendications**

**1.** Procédé de préparation d'une poudre d'oxyde mixte de silicium-titane pyrogène ayant

- une surface BET de 200 à 400 m$^2$/g,
- une teneur en dioxyde de silicium de 97,0 $\pm$ 1,5 % en poids,
- une teneur en dioxyde de titane de 3,5 $\pm$ 1,0 % en poids et
- la somme de la teneur en dioxyde de silicium et la teneur en dioxyde de titane est supérieure à 99,7 % en poids,

tous les pourcentages en poids par rapport à la quantité totale de la poudre, dans lequel

- 97,0 $\pm$ 1,5 parties en poids calculées en $SiO_2$ d'halogénure de silicium et 3,5 $\pm$ 1,5 parties en poids calculées en $TiO_2$ d'halogénure de titane sont évaporées, les vapeurs sont transférées dans une chambre de mélange,
- de l'hydrogène et de l'air primaire sont introduits dans la chambre de mélange séparément de celles-ci,
- le mélange des vapeurs d'halogénure de silicium et d'halogénure de titane, d'un gaz combustible contenant de l'hydrogène et de l'air primaire est ensuite allumé dans un brûleur et la flamme est brûlée dans une chambre de réaction,
- de l'air secondaire est en outre introduit dans la chambre de réaction, le solide est ensuite séparé des substances gazeuses, et
- le solide est ensuite libéré aussi loin que possible des substances contenant des halogénures par traitement avec de la vapeur d'eau à des températures de 250 à 700 °C **caractérisé en ce que**
- la quantité des substances requises constituées des chlorure de silicium, chlorure de titane, gaz combustible, air primaire et air secondaire est choisie de sorte qu'une température de flamme adiabatique $T_{ad}$ soit obtenue, pour laquelle ce qui suit est vrai :

$$900 °C < T_{ad} < 1200 °C,$$

avec
$T_{ad}$ = température des substances requises + somme des enthalpies de réaction des réactions partielles/capacité thermique des substances qui quittent la chambre de réaction, comprenant le dioxyde de silicium, l'eau, le chlorure d'hydrogène, le cas échéant le dioxyde de carbone, l'oxygène, l'azote et, le cas échéant, le gaz vecteur s'il ne s'agit pas de l'air ou de l'azote, la capacité thermique spécifique de ces substances à 1000 °C étant utilisée en tant que base.

**2.** Procédé selon la revendication 1,
**caractérisé en ce que**
$SiCl_4$ est utilisé en tant qu'halogénure de silicium, $TiCl_4$ est utilisé en tant qu'halogénure de titane et

$$T_{ad} = 1050 \pm 50 °C.$$

**3.** Procédé selon la revendication 1 ou 2,
**caractérisé en ce que**
la vitesse de sortie $v_{Br}$ des gaz depuis le brûleur dans l'espace de réaction est de 10 à 80 m/s.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 814058 A